# EUROPEAN PATENT APPLICATION

(11) **EP 4 792 816 A1**
(43) Date of publication of application: **19.08.2026**
(21) Application number: 25315046.0
(22) Date of filing: 12.02.2025
(51) Int. Cl.: A61K 31/7012, A61K 9/00, A61K 36/00, A61P 17/10

(54) **COSMETIC OR DERMATOLOGIC OR PHARMACEUTICAL COMPOSITION FOR USE IN THE TREATMENT AND/OR PREVENTION OF OILY OR OILY-PRONE SKIN AND/OR ACNE**

(71) Applicant: TS-Biotech, 92088 Paris La Défense Cedex (FR); Shandong Tiansheng Biotechnology CO., LTD., Weifang City 262605 (CN)
(72) Inventor: Junjie, Jiang, Weifang City, 262605 (CN); Yuhua, Jiana, Weifang City, 262605 (CN); Miao, Yu, Weifang City, 262605 (CN)
(74) Representative: Yes My Patent

(57) **Abstract**

The present invention relates to a cosmetic or dermatologic or pharmaceutical comprising an effective amount of a compound for use in the treatment and/or prevention of oily or oily-prone skin and/or acne, namely sialic acid and/or any derivatives thereof. The present invention further relates to non-therapeutical methods the treatment and/or prevention of oily or oily-prone skin and/or acne and uses of sialic acid and/or any derivatives thereof for inhibiting the expression of the 5alpha-reductase and for treating and/or preventing oily or oily-prone skin and/or acne and a use for treating and/or inhibiting *Cutibacterium acnes.*

## Description

The present invention relates to a cosmetic or dermatologic or pharmaceutical comprising an effective amount of a compound for use in the treatment and/or prevention of oily or oily-prone skin and/or acne, namely sialic acid and/or any derivatives thereof. The present invention further relates to non-therapeutical methods the treatment and/or prevention of oily or oily-prone skin and/or acne and uses of sialic acid and/or any derivatives thereof for inhibiting the expression of the 5alpha-reductase and for treating and/or preventing oily or oily-prone skin and/or acne and a use for treating and/or inhibiting *Cutibacterium acnes.*

Oily skin and acne are skin disorders affecting teenagers and adults, caused by excessive sebum secretion and excretion and hyperproliferation of *Cutibacterium acnes.* Such disorders are considered to provide aesthetic defaults or skin imperfections which need to be treated.

It has been discovered that 5alpha-reductase, a reduced coenzyme II (NADPH-)dependent membrane protease, is mainly involved in steroid metabolism. The function of 5alpha-reductase is to catalyze the conversion of testosterone to dihydrotestosterone (DHT) which causes many pathological changes such as acne when DHT accumulates to high levels. It is known that human 5alpha-reductase has two types of isoenzymes, namely type I and type II. Type I is mainly distributed in hair follicles, sebaceous gland, scalp, non-genital skin and liver and is a critical enzyme in skin androgen metabolism, while androgens can stimulate sebaceous glands. The 5alpha-reductase inhibitors finasteride and dutasteride commonly used in clinical practice have apparent side effects, such as psychological disorders, that need to be avoided for the health of the treated person. Thus, there is still a need to provide a novel and improved cosmetic or dermatologic or pharmaceutical composition for use in the treatment and/or prevention of oily or oily-prone skin and/or acne and/or alopecia.

According to the present invention, it has been found that sialic acid and/or any derivatives thereof permits to significantly reduce the expression of 5alpha-reductase and thus reduce the sebum secretion and/or acne while maintaining good hydration of the skin and not altering the barrier function of the skin. The reduction of 5alpha-reductase thanks to sialic acid and/or any derivatives thereof further permits to treat and/or prevent alopecia.

Therefore, the present invention relates to the use of sialic acid and/or any derivatives thereof for inhibiting the expression of the 5alpha-reductase. Indeed, the use of sialic acid and/or any derivatives permits to significantly reduce the expression of 5alpha-reductase and thus reduce the sebum secretion and/or acne while maintaining good hydration of the skin and not altering the barrier function of the skin. Further, the use of sialic acid and/or any derivatives thereof for inhibiting the expression of the 5alpha-reductase according to the present invention permits to significantly reduce the expression of 5alpha-reductase and thus reduce the sebum secretion linked to alopecia such that the sialic acid and/or any derivatives thereof and the composition of the present invention can be used for treating and/or preventing alopecia.

The present invention further relates to the use of sialic acid and/or any derivatives thereof for the treatment and/or prevention of oily or oily-prone skin and/or acne.

In view of the discussed needs, the present invention further relates to a cosmetic or dermatologic or pharmaceutical composition comprising:
an effective amount of a compound for use in the treatment and/or prevention of oily or oily-prone skin and/or acne, wherein the compound for use in the treatment and/or prevention of oily or oily-prone skin and/or acne is sialic acid and/or any derivatives thereof.

Preferably the effective amount of the compound for use in the treatment and/or prevention of oily or oily-prone skin and/or acne is of at least 0.01 weight-%, based on the weight of the cosmetic or dermatologic or pharmaceutical composition.

Preferably the compound for use in the treatment and/or prevention of oily or oily-prone skin and/or acne inhibits the expression of the 5alpha-reductase. Hence, sialic acid and/or any derivatives thereof is the compound for use in the treatment and/or prevention of oily or oily-prone skin and/or acne by inhibiting the expression of the 5alpha-reductase.

Preferably the compound for use in the treatment and/or prevention of oily or oily-prone skin and/or acne is sialic acid.

Optionally the compound for use in the treatment and/or prevention of oily or oily-prone skin and/or acne being sialic acid and/or any derivatives thereof is encapsulated or vectorized.

Preferably the cosmetic or dermatologic or pharmaceutical composition is free of cyclodextrin.

Preferably the derivatives of sialic acid are one or more of O-methyl sialic acid, O-acetyl sialic acid and N-glycolylneuraminic acid.

Preferably the derivatives of sialic acid are not sialyl sugars.

### Topical use

Preferably the cosmetic or dermatologic or pharmaceutical composition of the present invention is for topical use.

Preferably the effective amount of the compound for use in the treatment and/or prevention of oily or oily-prone skin and/or acne is in the range of from 0.01 to 10 weight-%, more preferably in the range of from 0.1 to 5 weight-%, more preferably in the range of from 0.25 to 1.5 weight-%, based on the weight of the cosmetic or dermatologic or pharmaceutical composition.

Preferably the cosmetic or dermatologic or pharmaceutical composition is in the form of a cream, an oil-in-water emulsion, a water-in-oil emulsion, a serum, an oil, a gel, an aqueous solution, a vectorization system, an anhydrous stick, a foam, a lotion, a milk, or a camouflage product, more preferably in the form of a cream, an oil-in-water emulsion or a water-in-oil emulsion.

Preferably the cosmetic or dermatologic or pharmaceutical composition further comprises a cosmetic or dermatologic or pharmaceutical acceptable carrier, more preferably the acceptable carrier is selected from the group consisting of water, glycerin, glycol, propanediol, and mixtures of two or more thereof, more preferably selected from the group consisting of water, glycerin, propanediol and mixtures of two or more thereof.

Preferably the cosmetic or dermatologic or pharmaceutical acceptable carrier is water.

Alternatively, preferably the cosmetic or dermatologic or pharmaceutical acceptable carrier comprises water and glycerin, wherein the weight ratio of glycerin to water is in the range of from 0.1:100 to 20:100.

Alternatively, preferably the cosmetic or dermatologic or pharmaceutical acceptable carrier comprises water and glycerin, wherein the weight ratio of propanediol to water is in the range of from 0.1:100 to 10:100.

Preferably the cosmetic or dermatologic or pharmaceutical composition further comprises one or more emulsifiers.

In the context of the present invention, any emulsifiers known in the art can be used as long as it plays its emulsifying role in the composition.

Preferably the content of emulsifier(s) in the cosmetic or dermatologic or pharmaceutical composition is in the range of from 0.1 to 10 wt.-%, more preferably in the range of from 0.2 to 7 wt.-%, more preferably in the range of from 0.3 to 5 wt.-%, based on the weight of the cosmetic or dermatologic or pharmaceutical composition.

Preferably the one or more emulsifiers are selected from the group consisting of cetearyl alcohol; ceteth-20; glyceryl stearate; steareth-20; a mixture of cetyl alcohol, glyceryl stearate, PEG-75 stearate, ceteth-20 and stearaeth-20; carbomer; xanthan gum; a mixture of sodium acrylate and sodium acryloyldimethyl taurate copolymer; a mixture of beheneth-30 phosphate, cetostearyl alcohol, dicetyl phosphate and cetyl phosphate; cetearyl olivate and sorbitan olivate; ceteareth-6 olivate and mixtures of two or more thereof; more preferably selected from the group consisting of cetearyl alcohol; ceteth-20; glyceryl stearate; steareth-20; a mixture of cetyl alcohol, glyceryl stearate, PEG-75 stearate, ceteth-20 and stearaeth-20; carbomer; xanthan gum; a mixture of sodium acrylate and sodium acryloyldimethyl taurate copolymer; a mixture of beheneth-30 phosphate, cetostearyl alcohol, dicetyl phosphate and cetyl phosphate; and mixtures of two or more thereof.

Preferably the cosmetic or dermatologic or pharmaceutical composition further comprises one or more emollients.

Preferably the emollient content in the cosmetic or dermatologic or pharmaceutical composition is in the range of from 0.25 to 20 wt.-%, more preferably in the range of from 0.5 to 15 wt.-%, more preferably in the range of from 0.75 to 12 wt.-%, based on the weight of the cosmetic or dermatologic or pharmaceutical composition.

Preferably the one or more emollients are selected from the group consisting of dicaprylyl carbonate; octyldodecanol; dicaprylyl ether; cyclomethicone; a mixture of isodecane, hydrogenated tetradecenyl and methylpentadecene; pentaerythrityl tetralaurate; isocetyl stearate; stearyl dimethicone; neopentyl glycol diethylhexanoate; cetyl ricinoleate; shorea robusta seed butter; and mixtures of two or more thereof; more preferably selected from the group consisting of dicaprylyl carbonate; octyldodecanol; dicaprylyl ether; cyclomethicone; a mixture of isodecane, hydrogenated tetradecenyl and methylpentadecene; pentaerythrityl tetralaurate; and mixtures of two or more thereof.

Preferably the cosmetic or dermatologic or pharmaceutical composition further comprises one or more of a pH regulator, a preservative, a hydrating agent, a thickening agent, a masking agent, an antioxidant, and a film-forming agent.

In the present invention, any pH regulator can be used for the cosmetic or dermatologic or pharmaceutical composition as long as it provides its pH regulator/stabilizer properties and is cosmetically/dermatologically/pharmaceutically acceptable. Examples of pH regulator are sodium hydroxide, sodium bicarbonate, potassium hydroxide, triethanolamine, and/or aminomethyl propanol. Preferably in the present invention, sodium hydroxide and/or sodium bicarbonate are used as pH regulator/buffering agent.

In the present invention, any preservatives can be used for the cosmetic or dermatologic or pharmaceutical composition as long as it provides its preservative properties and is cosmetically/dermatologically/pharmaceutically acceptable. Examples of a preservative are selected from the group consisting of phenoxyethanol; propylparaben; methylparaben; butylparaben; sorbic acid; benzoic acid; benzyl alcohol; and mixtures of two or more thereof.

In the present invention, any hydrating agents can be used for the cosmetic or dermatologic or pharmaceutical composition as long as it provides its hydrating properties and is cosmetically/dermatologically/pharmaceutically acceptable. Examples of a hydrating agent are a mixture of dimethicone, vinyl dimethicone cross polymer and silica.

In the present invention, any thickening agents can be used for the cosmetic or dermatologic or pharmaceutical composition as long as it provides its thickening properties and is cosmetically/dermatologically/pharmaceutically acceptable. Examples of a thickening agent are a mixture of polyacrylamide, C13-14 isoparaffin and laureth-7; a mixture of acrylates and C10-30 alkyl acrylate cross polymer; xanthan gum, Sodium carboxymethyl starch, and/ or a mixture of Pullulan (and) Sorbitol (and) Trehalose (and) Acacia Senegal Gum.

In the present invention, any masking agents can be used for the cosmetic or dermatologic or pharmaceutical composition as long as it provides its masking properties and is cosmetically/dermatologically/pharmaceutically acceptable. Examples of a masking agent are caprylic/capric triglyceride.

In the present invention, any film-forming agents can be used for the cosmetic or dermatologic or pharmaceutical composition as long as it provides its film-forming properties and is. cosmetically/dermatologically/pharmaceutically acceptable. Examples of a film-forming agent are biosaccharide gum-4, and/or hydroxypropyl starch.

In the present invention, any antioxidants can be used for the cosmetic or dermatologic or pharmaceutical composition as long as it provides its antioxidant properties and is cosmetically/dermatologically/pharmaceutically acceptable. Examples of an antioxidant are vitaminbased antioxidants such as tocopherol, ascorbic acid, retinol, beta-carotene; and/or zinc salt.

Optionally the cosmetic or dermatologic or pharmaceutical composition further comprises a plant-based ingredient, preferably the plant-based ingredient being one or more of green tea extract, *Desmodium* extract, nettle extract, Broccoli seed oil, chamomile extract, cucumber extract, and seaweed extract, more preferably the plant-based ingredient being one or more of green tea extract, *Desmodium* extract, nettle extract and broccoli seed oil.

Examples of seaweed extract are brown algae; red algae; green algae; blue-green algae (such as spirulina); and/or extracts of Laminariaceae, Laminariales laminariaceae, Undaria pinnatifida Suringar, Sargassum fusiforme, Gelidiaceae, Corallina, Palmata, Chondrus, Porphyra tenera, Ulvaceae, Ulva pertusaKjellman,Ascophyllum, Fucus, Nemacystus decipiens, Cladosiphon okamuranus, and Himanthalia.The above examples include extracts of hydrophytes such as Zostera marina L.

Preferably the cosmetic or dermatologic or pharmaceutical composition is an emulsion, more preferably an oil-in-water emulsion. Preferably the composition comprises, more preferably consist of, sialic acid as the compound for use in the treatment and/or prevention of oily or oily-prone skin and/or acne; octyldodecanol (as the emollient); cetearyl alcohol (as the emulsifier); caprylic/capric triglyceride (as a first masking agent); a mixture of polyacrylamide, C13-14 isoparaffin and laureth-7 (as the thickening agent); propylparaben (as the preservative); methylparaben (as a second preservative), sodium bicarbonate (as the pH regulator/buffer) and water as the cosmetic or dermatologic acceptable carrier.

Preferably the cosmetic or dermatologic or pharmaceutical composition is an emulsion, more preferably an oil-in-water emulsion. Preferably the composition comprises, more preferably consist of, sialic acid as the compound for use in the treatment and/or prevention of oily or oily-prone skin and/or acne; octyldodecanol, dicaprylyl carbonate, dicaprylyl ether and cyclomethiconel (as the emollients); carbomer and xhantan gum (as the emulsifiers); a mixture of cetyl alcohol, glyceryl stearate, PEG-75 stearate, ceteth-20 and stearaeth-20 (as a further emulsifier); a mixture of acrylates and C10-30 alkyl acrylate cross polymer (as the thickening agent); a mixture of phenoxyethanol, butylparaben and propylparaben (as the preservative); sodium hydroxide (as the pH regulator) and water and glycerin as the cosmetic or dermatologic acceptable carrier.

Preferably the cosmetic or dermatologic or pharmaceutical composition is an emulsion, more preferably an oil-in-water emulsion. Preferably the composition comprises, more preferably consist of, sialic acid as the compound for use in the treatment and/or prevention of oily or oily-prone skin and/or acne; pentaerythrityl tetralaurate, dicaprylyl carbonate and a mixture of isodecane, hydrogenated tetradecenyl and methylpentadecene (as the emollients); a mixture of beheneth-30 phosphate, cetostearyl alcohol, dicetyl phosphate and cetyl phosphate and a mixture of sodium acrylate and sodium acryloyldimethyl taurate copolymer (as the emulsifiers); phenoxyethanol (as the preservative); a mixture of dimethicone, vinyl dimethicone cross polymer and silica (as the hydrating agent); methylmethacrylate cross polymer (as the film-forming agent); sodium hydroxide (as the pH regulator) and water and propanediol as the cosmetic or dermatologic acceptable carrier.

Optionally, the cosmetic or dermatologic or pharmaceutical composition of the present invention further comprises one or more of flavors, pigments/dyes, antiseptics, skin care agents and UV-absorbing agent.

### Oral use

Preferably the cosmetic or dermatologic or pharmaceutical composition of the present invention is for oral use.

Preferably the effective amount of the compound for use in the treatment and/or prevention of oily or oily-prone skin and/or acne is in the range of from 2 to 100 weight-%, more preferably in the range of from 5 to 50 weight-%, more preferably in the range of from 5 to 40 weight-%, more preferably in the range of from 8 to 35 weight-%, based on the weight of the cosmetic or dermatologic or pharmaceutical composition.

Preferably the cosmetic or dermatologic or pharmaceutical composition is in the form of a liquid, a capsule, a gummy, a powder, or a gel.

Preferably the cosmetic or dermatologic or pharmaceutical composition further comprises a plant-based ingredient, preferably the plant-based ingredient being one or more of green tea extract, *Desmodium* extract, nettle extract, Broccoli seed oil, chamomile extract, cucumber extract, and seaweed extract, more preferably the plant-based ingredient being one or more of green tea extract, *Desmodium* extract, nettle extract and broccoli seed oil.

Examples of seaweed extract are brown algae; red algae; green algae; blue-green algae (such as spirulina); and/or extracts of Laminariaceae, Laminariales laminariaceae, Undaria pinnatifida Suringar, Sargassum fusiforme, Gelidiaceae, Corallina, Palmata, Chondrus, Porphyra tenera, Ulvaceae, Ulva pertusaKjellman,Ascophyllum, Fucus, Nemacystus decipiens, Cladosiphon okamuranus, and Himanthalia.The above examples include extracts of hydrophytes such as Zostera marina L.

Optionally, the cosmetic or dermatologic or pharmaceutical composition further comprises one or more of peptides, lipides, saccharides and polyphenols. Peptides may have lipidic chain(s) and/or sugar(s). Saccharides and lipides can have lipidic chain(s) or sugar(s), respectively. Further polyphenols can also comprise sugar(s) and/or lipidic chains.

Preferably the cosmetic or dermatologic or pharmaceutical composition further comprises an antioxidant and/or anti-inflammatory component, more preferably said component being selected from the group consisting of zinc, zinc bisglycinate, selenium, matcha tea, vitamin A, vitamin C, vitamin E, N-acetyl-cysteine, lutein, zeaxanthin, lycopene, turmeric, OPCs, OPC derivatives, glutathione, astaxanthin, and mixtures of two or more thereof, more preferably selected from the group consisting of zinc, zinc bisglycinate, selenium, matcha tea, and mixtures of two or more thereof.

Optionally the cosmetic or dermatologic or pharmaceutical composition further comprises minerals and/or vitamins, preferably one or more of vitamin D2, vitamin D3, and lactoferrine, more preferably vitamin D3 and lactoferrine.

Optionally the cosmetic or dermatologic or pharmaceutical composition comprises one or more excipients. Any excipients known by the skilled person can be used as long as it is adapted for dietary purpose.

Preferably the daily dosage of the compound for use in the treatment and/or prevention of oily or oily-prone skin and/or acne *(= the active ingredient)* is in the range of from 10 mg/day to 1g/day, more preferably in the range of from 50 mg/day to 750 mg/day, more preferably in the range of from 100 mg/day to 500 mg/day.

Preferably the cosmetic or dermatologic or pharmaceutical composition being for oral use comprises sialic acid as the active ingredient, broccoli seed oil, Desmodium extract, zinc bisglycinate, matcha tea and excipient(s). More preferably the content of sialic acid is in the range of from 5 to 20 weight-%, more preferably in the range of 10 to 18 weight-%, based on the weight of the composition.

Preferably the cosmetic or dermatologic or pharmaceutical composition being for oral use comprises sialic acid as the active ingredient, lactoferrine, zinc bisglycinate, vitamin D3 and selenium. More preferably the content of sialic acid is in the range of from 25 to 50 weight-%, more preferably in the range of 35 to 48 weight-%, based on the weight of the composition.

Preferably the cosmetic or dermatologic or pharmaceutical composition being for oral use comprises sialic acid as the active ingredient, nettle extract, green tea extract and selenium. More preferably the content of sialic acid is in the range of from 10 to 40 weight-%, more preferably in the range of 20 to 35 weight-%, based on the weight of the composition.

In the context of the present invention, in the cosmetic or dermatologic or pharmaceutical composition, namely for oral or topical use, the compound for use in the treatment and/or prevention of oily or oily-prone skin and/or acne, being sialic acid and/any derivatives thereof, is the sole effective ingredient for the treatment and/or prevention of oily or oily-prone skin and/or acne, being sialic acid and/any derivatives thereof.

Alternatively, in the cosmetic or dermatologic or pharmaceutical composition, namely for oral or topical use, the compound for use in the treatment and/or prevention of oily or oily-prone skin and/or acne, being sialic acid and/any derivatives thereof, is an effective ingredient for the treatment and/or prevention of oily or oily-prone skin and/or acne, being sialic acid and/any derivatives thereof which can be combined with co-active ingredients, such as salicylic acid, niacinamide, pyruvic acid, benzoyle peroxide, essential oil, retinoid, glycolic acid retinol, resorcinol, or any derivatives thereof.

The present invention further relates to a non-therapeutical method for the treatment and/or prevention of oily or oily-prone skin and/or acne, the method comprising
applying the cosmetic or dermatologic or pharmaceutical composition of the present invention to a skin in need at least once a day, preferably twice a day.

Preferably the application of the composition is repeated for a duration in the range of from 1 week to 6 months, more preferably in the range of from 2 weeks to 4 months.

The present invention further relates to a non-therapeutical method for the treatment and/or prevention of oily or oily-prone skin and/or acne, the method comprising
administering orally the cosmetic or dermatologic or pharmaceutical composition of the present invention to a person in need once or twice a day.

Preferably the administration of the composition is repeated for a duration in the range of from 1 week to 6 months, more preferably in the range of from 2 weeks to 4 months.

Preferably the daily dosage of the compound for use in the treatment and/or prevention of oily or oily-prone skin and/or acne (active ingredient) is in the range of from 10 mg/day to 1g/day, more preferably in the range of from 50 mg/day to 750 mg/day, more preferably in the range of from 100 mg/day to 500 mg/day.

In view of the discussed needs, the present invention further relates to a cosmetic or dermatologic or pharmaceutical composition comprising:
an effective amount of a compound for use in inhibiting the expression of the 5alpha-reductase,
wherein the compound for use in inhibiting the expression of the 5alpha-reductase is sialic acid and/or any derivatives thereof. Preferably the composition is for oral or topical use. Preferably the effective amount of the compound is as disclosed herein above relative to the other composition. According to the present invention, the ingredients of said composition and/or the proportions thereof are preferably as disclosed for the cosmetic or dermatologic or pharmaceutical composition which comprises the compound for use in the treatment and/or prevention of oily or oily-prone skin and/or acne disclosed herein.

The present invention further relates to the use of a cosmetic or dermatologic or pharmaceutical composition according to the present invention for the treatment and/or prevention of oily or oily-prone skin and/or acne.

The present invention further relates to the use of a cosmetic or dermatologic or pharmaceutical composition according to the present invention for inhibiting and/or treating *Cutibacterium acnes.*

The present invention is further illustrated by the following set of embodiments and combinations of embodiments resulting from the dependencies and back references as indicated. In particular, it should be noted that in each case where a range of embodiments is mentioned, for example in the context of a term such as "The composition of any one of embodiments 1 to 4", each embodiment in that range is intended to be explicitly disclosed to those skilled in the art, i.e., the wording of that term should be understood by those skilled in the art to be synonymous with "The composition of any one of embodiments 1, 2, 3 and 4". Furthermore, it is explicitly noted that the following set of embodiments represents a suitably structured part of the general description directed to preferred aspects of the present invention and, therefore, suitably supports, but does not represent, the claims of the present invention.

According to embodiment 1 of the present invention, a use of sialic acid and/or any derivatives thereof for inhibiting the expression of the 5alpha-reductase is defined.

Embodiment 2: Use of sialic acid and/or any derivatives thereof for the treatment and/or prevention of oily or oily-prone skin and/or acne.

Embodiment 3: Cosmetic or dermatologic or pharmaceutical composition comprising:
an effective amount of a compound for use in the treatment and/or prevention of oily or oily-prone skin and/or acne, wherein the compound for use in the treatment and/or prevention of oily or oily-prone skin and/or acne is sialic acid and/or any derivatives thereof.

Embodiment 4: The composition of embodiment 3, wherein the compound for use in the treatment and/or prevention of oily or oily-prone skin and/or acne inhibits the expression of the 5alpha-reductase.

Embodiment 5: The composition of embodiment 3 or 4, wherein the compound for use in the treatment and/or prevention of oily or oily-prone skin and/or acne is sialic acid.

Embodiment 6: The composition of any one of embodiments 3 to 5, being for topical use.

Embodiment 7: The composition of embodiment 6, wherein the effective amount of the compound for use in the treatment and/or prevention of oily or oily-prone skin and/or acne is in the range of from 0.01 to 10 weight-%, preferably in the range of from 0.1 to 5 weight-%, more preferably in the range of from 0.25 to 1.5 weight-%, based on the weight of the cosmetic or dermatologic or pharmaceutical composition.

Embodiment 8: The composition of embodiment 6 or 7, being in the form of a cream, an oil-in-water emulsion, a water-in-oil emulsion, a serum, an oil, a gel, an aqueous solution, a vectorization system, an anhydrous stick, a foam, a lotion, a milk, or a camouflage product, preferably in the form of a cream, an oil-in-water emulsion or a water-in-oil emulsion.

Embodiment 9: The composition of any one of embodiments 6 to 8, further comprising a cosmetic or dermatologic acceptable carrier, preferably the cosmetic or dermatologic acceptable carrier is selected from the group consisting of water, glycerin, glycol, propanediol, and mixtures of two or more thereof, more preferably selected from the group consisting of water, glycerin, propanediol and mixtures of two or more thereof.

Embodiment 10: The composition of any one of embodiments 3 to 9, further comprising one or more emulsifiers.

Embodiment 11: The composition of embodiment 10, wherein the one or more emulsifiers are selected from the group consisting of cetearyl alcohol; ceteth-20; glyceryl stearate; steareth-20; a mixture of cetyl alcohol, glyceryl stearate, PEG-75 stearate, ceteth-20 and stearaeth-20; carbomer; xanthan gum; a mixture of sodium acrylate and sodium acryloyldimethyl taurate copolymer; a mixture of beheneth-30 phosphate, cetostearyl alcohol, dicetyl phosphate and cetyl phosphate; cetearyl olivate and sorbitan olivate; ceteareth-6 olivate and mixtures of two or more thereof, preferably selected from the group consisting of cetearyl alcohol; ceteth-20; glyceryl stearate; steareth-20; a mixture of cetyl alcohol, glyceryl stearate, PEG-75 stearate, ceteth-20 and stearaeth-20; carbomer; xanthan gum; a mixture of sodium acrylate and sodium acryloyldimethyl taurate copolymer; a mixture of beheneth-30 phosphate, cetostearyl alcohol, dicetyl phosphate and cetyl phosphate; and mixtures of two or more thereof.

Embodiment 12: The composition of any one of embodiments 3 to 11, further comprising one or more emollients.

Embodiment 13: The composition of embodiment 12, wherein the one or more emollients are selected from the group consisting of dicaprylyl carbonate; octyldodecanol; dicaprylyl ether; cyclomethicone; a mixture of isodecane, hydrogenated tetradecenyl and methylpentadecene; pentaerythrityl tetralaurate; isocetyl stearate; stearyl dimethicone; neopentyl glycol diethylhexanoate; cetyl ricinoleate; shorea robusta seed butter; and mixtures of two or more thereof; preferably selected from the group consisting of dicaprylyl carbonate; octyldodecanol; dicaprylyl ether; cyclomethicone; a mixture of isodecane, hydrogenated tetradecenyl and methylpentadecene; pentaerythrityl tetralaurate; and mixtures of two or more thereof.

Embodiment 14: The composition of any one of embodiments 3 to 13, further comprising one or more of a pH regulator, a preservative, a hydrating agent, a thickening agent, a masking agent, an antioxidant, and a film-forming agent.

Embodiment 15: The composition of any one of embodiments 3 to 5, being for oral use.

Embodiment 16: The composition of embodiment 15, wherein the effective amount of the compound for use in the treatment and/or prevention of oily or oily-prone skin and/or acne is in the range of from 2 to 100 weight-%, preferably in the range of from 5 to 50 weight-%, more preferably in the range of from 5 to 40 weight-%, more preferably in the range of from 8 to 35 weight-%, based on the weight of the cosmetic or dermatologic or pharmaceutical composition.

Embodiment 17: The composition of embodiment 15 or 16, being in the form of a liquid, a capsule, a gummy, a powder, or a gel.

Embodiment 18: The composition of any one of embodiment 15 to 17, further comprising a plant-based ingredient, preferably the plant-based ingredient being one or more of green tea extract, *Desmodium* extract, nettle extract, Broccoli seed oil, chamomile extract, cucumber extract, and seaweed extract, preferably the plant-based ingredient being one or more of green tea extract, *Desmodium* extract, nettle extract and broccoli seed oil.

Embodiment 19: The composition of any one of embodiments 15 to 18, further comprising an antioxidant and/or anti-inflammatory component, preferably said component being selected from the group consisting of zinc, zinc bisglycinate, selenium, matcha tea, vitamin A, vitamin C, vitamin E, N-acetyl-cysteine, lutein, zeaxanthin, lycopene, turmeric, OPCs, OPC derivatives, glutathione, astaxanthin, and mixtures of two or more thereof, more preferably selected from the group consisting of zinc, zinc bisglycinate, selenium, matcha tea, and mixtures of two or more thereof.

Embodiment 20: The composition of any one of embodiments 15 to 19, further comprising minerals and/or vitamins, preferably one or more of vitamin D2, vitamin D3, and lactoferrine, more preferably vitamin D3 and lactoferrine.

Embodiment 21: The composition of any one of embodiments 15 to 20, wherein the daily dosage of the compound for use in the treatment and/or prevention of oily or oily-prone skin and/or acne *(active ingredient)* is in the range of from 10 mg/day to 1g/day, preferably in the range of from 50 mg/day to 750 mg/day, more preferably in the range of from 100 mg/day to 500 mg/day.

Embodiment 22: A non-therapeutical method for the treatment and/or prevention of oily or oily-prone skin and/or acne, the method comprising
applying the cosmetic or dermatologic or pharmaceutical composition of any one of embodiments 3 to 14 to a skin in need at least once a day, preferably twice a day.

Embodiment 23: The non-therapeutical method of embodiment 22, wherein the application of the composition is repeated for a duration in the range of from 1 week to 6 months, preferably in the range of from 2 weeks to 4 months.

Embodiment 24: A non-therapeutical method for the treatment and/or prevention of oily or oily-prone skin and/or acne, the method comprising
administering orally the cosmetic or dermatologic or pharmaceutical composition for use according to any one of embodiments 3 to 5 and 15 to 21 to a person in need once or twice a day.

Embodiment 25: The non-therapeutical method of embodiment 24, wherein the administration of the composition is repeated for a duration in the range of from 1 week to 6 months, preferably in the range of from 2 weeks to 4 months.

Embodiment 26: The non-therapeutical method of embodiment 24 or 25, wherein the daily dosage of the compound for use in the treatment and/or prevention of oily or oily-prone skin and/or acne (active ingredient) is in the range of from 10 mg/day to 1g/day, preferably in the range of from 50 mg/day to 750 mg/day, more preferably in the range of from 100 mg/day to 500 mg/day.

Embodiment 27: Use of the cosmetic or dermatologic or pharmaceutical composition according to any one of embodiments 3 to 21 for the treatment and/or prevention of oily or oily-prone skin and/or acne.

Embodiment 28: Use of a cosmetic or dermatologic or pharmaceutical composition according to any one of embodiments 3 to 21 for inhibiting and/or treating *Cutibacterium acnes.*

In the context of the present invention, the term "one or more of A, B and C" in an expression disclosing several possible features/possibilities, wherein each of A, B and C stands for specific features/possibilities, is to be understood as disclosing that explicitly either A, or B, or C, or A and B, or A and C, or B and C, or A and B and C. In this regard, it is noted that the skilled person is capable of transfer to the above abstract term to a concrete example.

The present invention is further illustrated by the following examples.

### EXAMPLES

### EXAMPLE 1 In-vitro testing: Study of the effect of sialic acid on the expression of 5-alpha-reductase in Hacat cells (oil control)

The aim of this test was to evaluate whether sialic acid had an effect on the 5alpha-reductase, namely a reduced coenzyme II (NADPH-)dependent membrane protease mainly involved in steroid metabolism. The function of 5alpha-reductase is to catalyze the conversion of testosterone to dihydrotestosterone which causes many pathological changes such as acne when DHT accumulates to high levels. Based on the results, it will be possible to evaluate whether sialic acid is effective for oil control activity (treating acne).

### I. Materials & Method

### I.1 Materials

### 1.1 Cell and cell culture

Hacat cells are human immortalized keratin-forming cells, they were cultured in high glucose Dulbecco's Modified Eagle Medium (DMEM), containing 10 wt.-% fetal bovine serum, 1 wt.-% penicillin-streptomycin, 5% CO₂ environment in a 37°C incubator.

### Equipment: CO₂ incubator; Leica^{®} DMi 8 inverted microscope; Applied Biosystems 7500 fast real time quantitative fluorescence PCR; Thermocycler Biometra TOne 96G PCR Instrument

### I.2 Method

### 2.1 Cell cultures (monolayer culture cells)

2x10⁶ Hacat cells were inoculated in a 6-well plate. When the cells were fused at about 70%, they were rinsed twice with phosphate buffer saline (PBS), 2 ml DMEN was added to cover the Hacat cells, the blank control group was left untreated, 5 µM (micromolar) Finasteride (a known 5alpha-reductase inhibitor which can be used for treating acne) was added to the positive control group, and a concentration of 0.0125 wt.-% of sialic acid and a concentration of 0.05 wt.-% of sialic acid was added respectively to the two experimental groups. The incubation continued for 48h at 37 °C in the incubator.

### 2.2 RNA Extraction

After discarding the medium from the monolayer culture cells, the cells were rinsed one time with PBS, lysed in the culture plate by adding 1mL TRIzol, and pipetted several times. The homogenized samples were left at room temperature (15-30°C) for 5 min to allow complete separation of the nucleic acid-protein complexes. 0.2 ml of chloroform per 1 ml of TRIzol was added, the sample was checked vigorously for 15 seconds and left at room temperature for 3 min, centrifuged for 15 min at 2-8°C (at 10,000 ×g). The upper aqueous phase was transferred to a new tube and the RNA was precipitated in the aqueous phase with isopropanol: 0.5 ml of isopropanol per 1 ml of TRIzol was added, the sample was left at room temperature for 10 min, centrifuged for 10 min at 2-8°C (at 10,000 ×g). A gelatinous precipitate appears on the side and bottom of the tube after the centrifugation. The supernatant was removed and the RNA residue washed with 75% ethanol: addition of at least 1ml of 75% ethanol solution followed by centrifugation for 5 min at 2-8°C at no more than 7,500 ×g, then the supernatant was discarded. The RNA precipitate was then dried at room temperature and allowed to dry naturally for 10 min on an ultra-clean table. Finally, 25 µl (microliter) of RNase(ribonuclease)-free water was added for dissolution.

### 2.3 cDNA (complementary DNA) synthesis was performed using NovoScript RT kit

| No. | Reactants | Volume |
|---|---|---|
| 1 | 5 x NovoscriptRT Buffer | 4 µl |
| 2 | pd (N) 6 (random hexamer primers) | 1 µl |
| 3 | dNTP | 2 µl |
| 4 | Novoscript RT Reverse Transcriptase | 1 µl |
| 5 | DEPC (diethylpyrocarbonate) H₂O | 10 µl |
| 6 | RNA templates | 2 µg |
| 7 | Tota Volume | 20 µl |

The entire procedure was performed on ice with the solution mixed by flicking the bottom of the tube and centrifuged at 6000 rpm at 25°C for 5 min and, at 42°C for 30 min then stored at 4°C. cDNA was then used for qPCR.

### 2.4 qPCR (quantitative polymerase chain reaction): The PCR assay was performed using the NovoScript SYBR qPCR SuperMix Plus kit. The reaction system was configured as follows.

| No. | Reactants | Volume |
|---|---|---|
| 1 | 2 x NovoScript SYBR qPCR SuperMix Plus | 10 µl |
| 2 | Primer F (10 µM) | 0.4 µl |
| | DNA seq.: CTACGGGCATCGGTGCTTA | |
| 3 | Primer R (10 µM) | 0.4 µl |
| | DNA seq.: AATCGCCATTGTACACGCCA | |
| 4 | ROX (Rhodamine X) | 0.4 µl |
| 5 | DEPC H₂O | 6.8 µl |
| 6 | cDNA | 2 µg |
| 7 | Tota Volume | 20 µl |

Flick the bottom of the tube to mix the above solutions and centrifuge briefly. A qPCR assay was performed, and the assay results were achieved using the 2^{-ΔΔCt} method for the calculation of relative expression levels. The data were statistically processed by the GraphPad Prism 8.0 software and all experimental data were expressed as *x̅* ± SD.

### I.3 Results

**Table 1 Effect of sialic acid on Hacat cell 5alpha-reductase**

| Experimental Group | Blank Control | Positive Control (Finasteride 5µM) | Sialic Acid (0.0125 % w/v) | Sialic Acid (0.05 % w/v) |
|---|---|---|---|---|
| Relative expression of 5 alpha-reductase (%) | 100 ± 8% | 37.66 ± 6.01% | 48.14 ± 11.23%* | 10.86 ± 8.13%**^{#} |

| | | | | |
|---|---|---|---|---|
| (Compared with blank control, *p<0.05, **p<0.01; compared with positive control, ^{#}p<0.05) *X % w*/*v sialic acid = X g of sialic acid in 100 ml culture medium (solution)* | | | | |

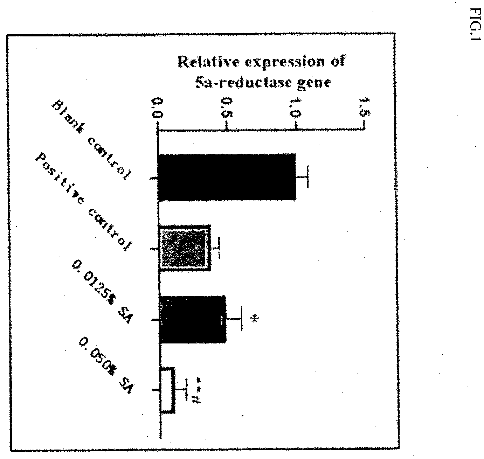

As may be taken from Table 1 and FIG. 1, the expression of 5alpha-reductase in the experimental groups using sialic acid is significantly reduced compared with the blank control group, and the differences were all statistically relevant as p<0.05. Further, it is noted that the expression of 5alpha-reductase in the experimental groups using sialic acid at a concentration of 0.05 % w/v is significantly lower than that of the positive control with p<0.05. Therefore, based on these experiments, one could conclude that sialic acid significantly reduces the expression of 5alpha-reductase in cells at different concentrations and thus is usable in composition for controlling oily skin/oily-prone skin/acne.

### EXAMPLE 2 In-vivo testing: Study of the effect of a cosmetic or dermatologic or pharmaceutical composition according to the present invention on oily skin with acne

### I. Formulation of the tested compositions

**Table 1 Emulsion**

| **Phase** | **INCI Name** | **Inventive Product** | **Comparative Product (Vehicle or Placebo)** |
|---|---|---|---|
| A | Octyldodecanol | 1 | 1 |
| | Cetearyl Alcohol | 0.5 | 0.5 |
| | Caprylic/Capric Triglyceride | 3.5 | 3.5 |
| | Polyacrylamide (45%), C13-14 Isoparaffin (25%), Laureth-7 (8%), Aqua (22%) | 1.5 | 1.5 |
| | Propylparaben | 0.03 | 0.03 |
| B | Water | q.s. 100 | q.s. 100 |
| | Methylparaben | 0.02 | 0.02 |
| C | **Acetylneuraminic Acid** (Sialic Acid) | **0.5** | **0** |
| | Sodium Bicarbonate | q.s. pH 5 | q.s. pH 5 |
| | Water | 10 | 10 |

| | | | |
|---|---|---|---|
| Protocol: Prepare Phase A by mixing the ingredients at 75°C with vigorous agitation. Add the premixed phase C components. Then, add the phase B components. Readjust pH by adding sodium bicarbonate. | | | |

### II. Experimental protocol

### II.1 Panel & tested parameters

Panel: 2 groups of 32 participants (long time acne subjects) each, one group was treated with the inventive product and one group with placebo (control group). Then, the sample was distributed to the participants on D0, the participants were required to apply it for 4 consecutive weeks in accordance with the sample application method, namely *apply the sample on face and massage gently until it is fully absorbed + apply it once in the morning and once in the evening,* and to apply the placebo cream for one week. Facial images of the participants were collected by researchers, the skin indexes were collected and the participants conducted the questionnaire survey on D0 (before sample use), D14 (14 days after sample use), D28 (28 days after sample use), D35 (discontinue the tested cream (inventive cream or placebo depending on the group) and use the placebo for 7 days), to evaluate the sample's anti-acne, repairing, oil control, soothing effects, as well as its abilities to reduce spots, improve skin glossiness and tighten pores through the statistical test. Test type: Double-blind controlled test. Comparison Method: Self-Comparison

The following parameters were observed:

**Table 2**

| **Parameters** | **Apparatus** | **Parameter interpretation** | **Target** |
|---|---|---|---|
| Sebum - T zone | Sebumeter^{®} SM815* | The smaller the measurement value, the less oil content | Oil-control |
| Acne volume | Antera 3D^{®}** (Miravex) | The smaller the measured value, the less the acne volume | Acne |
| Acne area | Antera 3D^{®}** (Miravex) | The smaller the measured value, the less the acne area | Acne |
| Pore Quantity | Visia^{®} Gen 7*** (Canfield) | The larger the measurement value, the more pronounced/visible the pore | Pore |
| Porphyrin number . | Visia^{®} Gen 7*** (Canfield) | The smaller the value, the less bacterial secretions in the blackhead | *Cutibacterium acnes* |
| Stratum Corneum Hydration | Corneometer CM825**** | The larger the measurement value, the higher the skin hydration | Skin hydration |
| Trans Epidermal Water Loss (TEWL) | Tewameter^{®} TM Hex***** | The lower the measurement value, the higher the skin barrier function . | Skin barrier |

| | | | |
|---|---|---|---|
| * The sebumeter method is used for skin oil testing. Based on the principle of photometer, the special matte tape will become a semi-transparent tape after absorbing the oil on the human skin and its light transmittance will change. The more oil you absorb, the more light you transmit. ** The apparatus is used to capture local facial images of the participants. The instrument's LED lights emit seven wavelengths of visible light to irradiate the skin from different angles, obtaining local skin images. Computer-aided methods are employed to simulate and reconstruct the three-dimensional structure of the skin surface and the distribution of skin chromophores. This can be used to analyze the surface morphology of the skin (such as the volume of skin surface depressions and protrusions), the degree of skin melanin, erythema and uniformity, skin color, skin texture and roughness. The calculation of acne volume and acne area is based on the volume of skin protrusions *** The standard light sources are used to produce images of and pores. The pores were detected by identifying very small spots and their color was darker than the normal skin. UV-light is used to produce ultraviolet stain images and porphyrin images. Porphyrins fluoresce at exposure to ultraviolet light. Porphyrins were produced by bacteria in the skin, specifically *Cutibacterium acnes.* ****The skin moisture is measured based on the internationally recognized capacitance method. As the hydration of the stratum corneum increases, its dielectric properties change. The dielectric constant of water is higher than that of most other substances. *****Water is constantly evaporating from the skin which is a part of the important body's metabolism. The Trans Epidermal Water Loss is measured by a probe constituted by sensors of relative humidity and temperature. | | | |

### II.2 Results

**Table 3 Descriptive statistics of oil contents (Sebumeter)**

| Descriptive statistics | Oil contents, unit: a.u. | | | | | | | |
|---|---|---|---|---|---|---|---|---|
| | Product group | | | | Placebo control group | | | |
| | D0 | D14 | D28 | D35 | D0 | D14 | D28 | D35 |
| Mean | 57.13 | 46.44 | 43.91 | 45.38 | 57.53 | 63.59 | 55.78 | 54.38 |
| Standard deviation | 42.45 | 34.29 | 24.68 | 38.43 | 33.89 | 30.63 | 35.92 | 38.95 |
| Standard error | 7.62 | 6.16 | 4.43 | 6.90 | 6.09 | 5.50 | 6.45 | 7.00 |
| Maximum | 220.00 | 208.00 | 133.00 | 173.00 | 166.00 | 142.00 | 131.00 | 188.00 |
| Minimum | 8.00 | 12.00 | 4.00 | 8.00 | 16.00 | 5.00 | 4.00 | 8.00 |
| Median | 47.50 | 43.50 | 39.50 | 36.00 | 48.50 | 59.50 | 43.00 | 43.00 |
| Change rate (vs D0) | / | -18.71% | -23.14% | -20.57% | / | 10.53% | -3.04% | -5.48% |

$Change rate \left(%\right) = \left(Mean after use-Mean before use\right) / Mean before use * 100$

After the participants used the sample for 14 days, the forehead **oil content** was significantly improved by 18.71% (p=0:015) compared to that before sample use; and the oil content was also significantly improved (p=0.0039) compared to the placebo group. At D28, the forehead oil content was significantly improved by 23.14% (p=0.015) compared to that before sample use; however, there was no significant improvement compared to the placebo group. After the participants discontinue the sample and use the placebo for 7 days, the forehead oil contents are significantly reduced by 20.57% (p=0.006) compared to that before sample use; however, there was no significant improvement compared to the placebo group. Thus, according to these results, it is concluded that the composition of the present invention (sialic acid) has **oil control efficiency (sebum regulation).**

**Table 4 Descriptive statistics of acne volume**

| Descriptive statistics | Acne volume, unit: mm³ | | | | | | | |
|---|---|---|---|---|---|---|---|---|
| | Product group | | | | Placebo control group | | | |
| | D0 | D14 | D28 | D35 | D0 | D14 | D28 | D35 |
| Mean | 1.044 | 0.526 | 0.213 | 0.125 | 0.742 | 0.662 | 0.287 | 0.174 |
| Standard deviation | 0.451 | 0.349 | 0.208 | 0.163 | 0.291 | 0.263 | 0.258 | 0.141 |
| Standard error | 0.081 | 0.063 | 0.037 | 0.029 | 0.052 | 0.047 | 0.046 | 0.025 |
| Maximum | 1.897 | 1.232 | 0.984 | 0.652 | 1.755 | 1.435 | 1.010 | 0.725 |
| Minimum | 0.340 | 0.000 | 0.000 | 0.000 | 0.253 | 0.168 | 0.002 | 0.028 |
| Median | 0.926 | 0.492 | 0.173 | 0.055 | 0.728 | 0.627 | 0.191 | 0.117 |
| Change rate (vs D0) | / | -49.62% | -79.60% | -88.03% | / | -10.78% | -61.32% | -76.55% |

$Change rate \left(%\right) = \left(Mean after use-Mean before use\right) / Mean before use * 100$

After the participants use the sample for 14 days, the **acne volume** is significantly reduced by 49.62%(p<0.001) compared to that before sample use and the acne volume is significantly reduced (p<0.001) compared to the placebo control group. After the participants use the sample for 28 days, the acne volume is significantly reduced by 79.60%(p<0.001) compared to that before sample use; and the acne volume is significantly reduced (p=0.027) compared to the placebo control group. After the participants discontinue the sample and use the placebo for 7 days, the acne volume is significantly reduced by 88.03%(p<0.001) compared to that before sample use; and the acne volume is significantly reduced (p<0.001), and the change rate of acne volume is significantly improved (p=0.007) compared to the placebo control group. Thus, according to these results, it is concluded that the composition of the present invention (sialic acid) has **anti-acne efficiency.**

**Table 5 Descriptive statistics of acne area**

| Descriptive statistics | Acne area unit: mm² | | | | | | | |
|---|---|---|---|---|---|---|---|---|
| | Product group | | | | Placebo control group | | | |
| | D0 | D14 | D28 | D35 | D0 | D14 | D28 | D35 |
| Mean | 7.98 | 5.20 | 2.03 | 1.49 | 6.54 | 4.86 | 3.10 | 2.05 |
| Standard deviation | 3.69 | 3.48 | 2.14 | 1.73 | 2.71 | 2.96 | 2.51 | 1.85 |
| Standard error | 0.66 | 0.63 | 0.38 | 0.31 | 0.49 | 0.53 | 0.45 | 0.33 |
| Maximum | 18.46 | 12.32 | 9.84 | 6.52 | 13.55 | 13.35 | 10.10 | 7.25 |
| Minimum | 2.57 | 0.00 | 0.00 | 0.00 | 2.17 | 0.51 | 0.14 | 0.11 |
| Median | 8.12 | 4.66 | 1.69 | 0.77 | 6.19 | 4.49 | 2.55 | 1.17 |
| Change rate (vs D0) | / | -34.84% | -74.56% | -81.33% | / | -25.69% | -52.60% | -68.65% |

$Change rate \left(%\right) = \left(Mean after use-Mean before use\right) / Mean before use * 100$

After the participants use the sample for 14 days, the **acne area** is significantly reduced by 34.84% (p<0.001) compared to that before sample use; however, compared to the placebo control group there is no significant reduction. After the participants use the sample for 28 days, the acne area is significantly reduced by 74.56% (p<0.001) compared to that before sample use; and the acne area is also significantly reduced (p=0.001) compared to the placebo control group. After the participants discontinue the sample and use the placebo for 7 days, the acne area is significantly reduced by 81.33% (p<0.001) compared to that before sample use; and the acne area is also significantly reduced (p=0.015) compared to the placebo control group. Thus, according to these results, it is concluded that the composition of the present invention (sialic acid) has **anti-acne efficiency.**

**Table 6 Descriptive statistics of pore quantity**

| Descriptive statistics | Pores Quantity, Unit: pcs | | | | | | | |
|---|---|---|---|---|---|---|---|---|
| | Product group | | | | Placebo control group | | | |
| | D0 | D14 | D28 | D35 | D0 | D14 | D28 | D35 |
| Mean | 1483.1 | 1260.6 | 1239.8 | 1187.3 | 1416.9 | 1299.5 | 1282.2 | 1266.7 |
| Standard deviation | 650.0 | 520.3 | 476.7 | 508.2 | 685.0 | 722.0 | 673.0 | 632.4 |
| Standard error | 116.7 | 93.4 | 85.6 | 91.3 | 123.0 | 129.7 | 120.9 | 113.6 |
| Maximum | 3651 | 2672 | 2293 | 2353 | 3127 | 3145 | 2895 | 3035 |
| Minimum | 370 | 325 | 436 | 236 | 359 | 358 | 395 | 380 |
| Median | 1507 | 1242 | 1154 | 1245 | 1424 | 1119 | 1203 | 1135 |
| Change rate (vs D0) | / | -15.00% | -16.40% | -19.94% | / | -8.29% | -9.51% | -10.60% |

$Change rate \left(%\right) = \left(Mean after use-Mean before use\right) / Mean before use * 100$

After the participants use the sample for 14 days, the Pores Quantity significantly reduced by 15.00% compared to that before sample use (p<0.001); however, there is no significant decrease for the Pores Quantity compared to the placebo control group. After the participants use the sample for 28 days, the Pores Quantity is significantly reduced by 16.40% (p<0.001) compared to that before sample use; however, there is no significant decrease on the Pores Quantity compared to the placebo control group. After the participants discontinue the sample and use the placebo for 7 days, the Pores Quantity is significantly reduced by 19.94%(p<0.001) compared to that before sample use; and the Pores Quantity is also significantly reduced (p=0.007) compared to the placebo control group. Thus, according to these results, it is concluded that the composition of the present invention (sialic acid) permits to **tighten skin pores**.

**Table 7 Descriptive statistics of porphyrin number**

| Descriptive statistics | Number of porphyrins. Unit: pcs | | | | | | | |
|---|---|---|---|---|---|---|---|---|
| | Product group | | | | Placebo control group | | | |
| | D0 | D14 | D28 | D35 | D0 | D14 | D28 | D35 |
| Mean | 3132.4 | 2391.8 | 2588.6 | 2687.8 | 3177.6 | 3150.3 | 3125.5 | 3133.0 |
| Standard deviation | 1826.1 | 1603.6 | 1576.4 | 1622.9 | 1717.9 | 1896.9 | 1882.3 | 1822.7 |
| Standard error | 328.0 | 288.0 | 283.1 | 291.5 | 308.5 | 340.7 | 338.1 | 327.4 |
| Maximum | 7947 | 7060 | 6602 | 6908 | 7769 | 7809 | 7773 | 6825 |
| Minimum | 113 | 246 | 296 | 376 | 445 | 283 | 209 | 418 |
| Median | 2773 | 1904 | 2298 | 2400 | 3083 | 2920 | 3199 | 3043 |
| Change rate (vs D0) | / | -23.64% | -17.36% | -14.19% | / | -0.86% | -1.64% | -1.40% |

$Change rate \left(%\right) = \left(Mean after use-Mean before use\right) / Mean before use * 100$

After the participants use the sample for 14 days, the number of Porphyrin significantly reduces by 23.64% compared to that before sample use (p<0.001); the number of porphyrins is significantly reduced (p=0.011) and the change rate of number of porphyrins is significantly improved (p=0.034) compared to the placebo control group. After the participants use the sample for 28 days, the number of porphyrins is significantly reduced by 17.36% (p<0.001) compared to that before sample use; and the number of porphyrins is significantly reduced (p=0.01 1) compared to the placebo control group. After the participants discontinue the sample and use the placebo for 7 days, the number of porphyrins is significantly reduced by 14.19%(p<0.001) compared to that before sample use; the number of porphyrins is also significantly reduced (p=0.018) and the change rate of number of porphyrins is significantly improved (p=0.007) compared to the placebo control group. Thus, according to these results, it is concluded that the composition of the present invention (sialic acid) permits to **reduce sebaceous gland content of *Cutibacterium acnes*** and thus **acne.**

**Table 8 Descriptive statistics of Stratum Corneum hydration**

| | | | | | | | | |
|---|---|---|---|---|---|---|---|---|
| Descriptive statistics | Stratum Corneum Hydration, unit: a.u. | | | | | | | |
| | Product group | | | | Placebo control group | | | |
| | D0 | D14 | D28 | D35 | D0 | D14 | D28 | D35 |
| Mean | 55.27 | 62.59 | 64.51 | 66.65 | 54.97 | 59.06 | 58.04 | 60.62 |
| Standard deviation | 13.31 | 13.96 | 13.90 | 11.41 | 14.32 | 13.80 | 14.07 | 14.27 |
| Standard error | 2.39 | 2.51 | 2.50 | 2.05 | 2.57 | 2.48 | 2.53 | 2.56 |
| Maximum | 75.60 | 80.73 | 88.60 | 86.07 | 77.70 | 77.90 | 84.17 | 83.27 |
| Minimum | 20.30 | 16.43 | 14.73 | 30.43 | 25.57 | 28.80 | 22.40 | 24.83 |
| Median | 56.49 | 64.23 | 67.17 | 68.67 | 53.63 | 63.60 | 60.64 | 61.77 |
| Change rate (vs D0) | / | 13.24% | 16.72% | 20.59% | / | 7.44% | 5.58% | 10.28% |

$Change rate \left(%\right) = \left(Mean after use-Mean before use\right) / Mean before use * 100$

After the participants use the sample for 14 days (D14), the Stratum Corneum Hydration is significantly improved(p<0.001) by 13.24 % compared to at D0 (before the test); however, no particular improvement has been found compared to the placebo group. Further, at D28, the Stratum Corneum Hydration is significantly improved (p<0.001) by 16.72% compared to at D0; and also significantly improved(p=0.047) compared to the placebo control group. After the participants discontinue the sample and use the placebo for 7 days, the Stratum Corneum Hydration is significantly improved (p = 0.035), the change rate of stratum corneum hydration is significantly improved (p=0.037) compared to the placebo control group. Thus, according to these results, it is concluded that the composition of the present invention (sialic acid) shows **moisturizing efficiency.**

**Table 9 Descriptive statistics of trans-epidermal water loss (TEWL)**

| Descriptive statistics | Trans Epidermal Water Loss (TEWL). unit: g/h/m² | | | | | | | |
|---|---|---|---|---|---|---|---|---|
| | Product group | | | | Placebo control group | | | |
| | D0 | D14 | D28 | D35 | D0 | D14 | D28 | D35 |
| Mean | 20.19 | 17.72 | 18.09 | 18.15 | 21.31 | 19.90 | 19.51 | 20.36 |
| Standard deviation | 3.69 | 3.69 | 3.19 | 3.70 | 5.17 | 5.20 | 4.74 | 5.45 |
| Standard error | 0.66 | 0.66 | 0.57 | 0.66 | 0.93 | 0.93 | 0.85 | 0.98 |
| Maximum | 26:66 | 24.66 | 26.38 | 27.81 | 35.76 | 35.35 | 31.77 | 34.71 |
| Minimum | 15.30 | 11.56 | 12.64 | 11.13 | 15.32 | 9.40 | 11.84 | 10.04 |
| Median | 19.44 | 17.09 | 17.97 | 17.25 | 20.09 | 19.10 | 19.16 | 21.10 |
| Change rate (vs D0) | / | -12.23% | -10.40% | -10.10% | / | -6.62% | -8.45% | -4.46% |

$Change rate \left(%\right) = \left(Mean after use-Mean before use\right) / Mean before use * 100$

After the participants use the sample for 14 days, the Trans Epidermal Water Loss (TEWL) significantly reduces by 12.23% (p<0.001) compared to that before sample use; the Trans Epidermal Water Loss (TEWL) is also significantly improved (p=0.026) compared to the placebo control group. After the participants use the sample for 28 days, the Trans Epidermal Water Loss (TEWL) significantly reduces by 10.40% (p<0.001) compared to that before sample use; however, there was no significant improvement compared to the placebo control group. After the participants discontinue the sample and use the placebo for 7 days, the Trans Epidermal Water Loss (TEWL) significantly reduces by 10.10% (p=0.001) compared to that before sample use; the Trans Epidermal Water Loss (TEWL) was not significantly improved compared to the placebo control group. The diminution of significative effect over time can be explained by the fact that the TEWL was greatly reduced such that the significance of the values is lowered. This, however, does not impact on the results of the testing of the composition of the present invention (sialic acid) for TEWL which permits to conclude that the skin barrier is maintained or even improved while acne is diminished as illustrated by the other tests.

### EXAMPLE 3 Formulations for oral use

### 3.1 Formula A: Anti-acne and detox powder (2g of powder to dilute)

### Dosage: 2g of powder/day

| Ingredients | Dose | Daily dose |
|---|---|---|
| Sialic acid | 300 mg | 300 mg |
| Broccoli seed oil | 200 mg | 200 mg |
| *Desmodium* extract | 50 mg | 50 mg |
| Zinc bisglycinate | 50 mg | 50 mg |
| Matcha tea | 1000 mg | 1000 mg |
| Excipients | Qsp (2g) | |

### 3.2 Formula B: Anti-acne/anti-inflammation capsule (300 mg capsule)

### Dosage: 2 capsules/day

| Ingredients | Dose/capsule | Daily dose |
|---|---|---|
| Sialic acid | 100 mg | 200 mg |
| Lactoferrin | 100 mg | 200 mg |
| Zinc bisglycinate | 25 mg | 50 mg |
| Vitamin D3 | 0.013 mg | 0.026 mg |
| Selenium (Se) | 0.028 mg | 0.056 mg |

### 3.3 Formula C: Anti-acne/sebum control gummy (1g/gummy)

### Dosage: 2 gummies/day

| Ingredients | Dose/gummy | Daily dose |
|---|---|---|
| Sialic acid | 50 mg | 100 mg |
| Nettle extract | 100 mg | 200 mg |
| Green tea extract | 25 mg | 50 mg |
| Selenium (Se) | 0.028 mg | 0.056 mg |

### EXAMPLE 4: Formulations for skin products (topical use)

### 4.1 Emulsion for acne skin

| Ingredients - INCI Name | Wt.-% |
|---|---|
| ***PHASE A*** | |
| Cetyl alcohol / glyceryl stearate / PEG-75 stearate / ceteth-20 / steareth-20 | 3.00 |
| Dicaprylyl carbonate | 3.00 |
| Octyldodecanol | 3.00 |
| Dicaprylyl ether | 3.00 |

| ***PHASE B*** | |
|---|---|
| Aqua | q.s. 100 |
| Phenoxyethanol / butylparaben / propylparaben | 0.50 |
| Carbomer | 0.10 |
| Acrylates/C10-30 alkyl acrylate crosspolymer | 0.10 |
| Glycerin | 2.00 |
| Xanthan gum | 0.20 |

| ***PHASE C*** | |
|---|---|
| Sodium hydroxide | q.s. pH 5.5 - 6.0 |
| Acetylneuraminic acid | 0.5 |
| Cyclomethicone | 1.00 |

| | |
|---|---|
| Protocol: Prepare Phase A by mixing the ingredients at 75°C. Prepare Phase B by mixing the ingredients at 75°C. Create an emulsion by pouring B into A with vigorous agitation. Further, add the premixed phase C components and Readjust pH to 5.5-6. | |

### 4.2 Emulsion for acne-prone skin

| Ingredients - INCI Name | Wt.-% |
|---|---|
| ***PHASE A*** | |
| Aqua | q.s. 100 |
| Sodium hydroxide | q.s. pH 5.5 - 6.0 |
| Acetylneuraminic acid | 0.5 |
| Phenoxyethanol | 0.90 |

| ***PHASE B*** | |
|---|---|
| Beheneth-30 phosphate / Cetostearyl alcohol/ Dicetyl phosphate/ Cetyl phosphate | 1.80 |
| Dicaprylyl carbonate | 4.00 |
| Isodecane / Hydrogenated tetradecenyl / Methylpentadecene | 4.00 |
| Pentaerythrityl tetralaurate | 2.00 |
| Dimethicone / vinyl dimethicone cross polymer (and) silica | 4.00 |

| ***PHASE C*** | |
|---|---|
| Methylmethacrylate cross polymer | 2.00 |
| Sodium hydroxide | q.s. pH 5.5-6 |
| | |

| ***PHASE D*** | |
|---|---|
| Propanediol | 2.00 |
| Sodium acrylate / Sodium acryloyldimethyl taurate copolymer | 1.50 |

| | |
|---|---|
| Protocol: Prepare Phase A by mixing the ingredients at 75°C. Prepare Phase B by mixing the ingredients at 75°C. Create the emulsion by pouring B into A with vigorous agitation. Add phase C (including the readjustment of the pH with sodium hydroxide in said phase). At 50°C, add the premixed phase D components. | |

### DESCRIPTION OF THE FIGURES

- FIG. 1: shows the effect of sialic acid (SA) on the expression of Hacat cell 5alpha-reductase. (Compared with blank control, *p<0.05, **p<0.01; compared with positive control, ^{#}p<0.05)

## Claims

1. Use of sialic acid and/or any derivatives thereof for inhibiting the expression of the 5alpha-reductase.

2. Cosmetic or dermatologic or pharmaceutical composition comprising:
an effective amount of a compound for use in the treatment and/or prevention of oily or oily-prone skin and/or acne, wherein the compound for use in the treatment and/or prevention of oily or oily-prone skin and/or acne is sialic acid and/or any derivatives thereof.

3. The composition of claim 2, wherein the compound for use in the treatment and/or prevention of oily or oily-prone skin and/or acne inhibits the expression of the 5alpha-reductase.

4. The composition of claim 2 or 3, wherein the compound for use in the treatment and/or prevention of oily or oily-prone skin and/or acne is sialic acid.

5. The composition of any one of claims 2 to 4, being for topical use; preferably the cosmetic or dermatologic or pharmaceutical composition is in the form of a cream, an oil-in-water emulsion, a water-in-oil emulsion, a serum, an oil, a gel, an aqueous solution, a vectorization system, an anhydrous stick, a foam, a lotion, a milk, or a camouflage product, more preferably in the form of a cream, an oil-in-water emulsion or a water-in-oil emulsion.

6. The composition of claim 5, wherein the effective amount of the compound for use in the treatment and/or prevention of oily or oily-prone skin and/or acne is in the range of from 0.01 to 10 weight-%, preferably in the range of from 0.1 to 5 weight-%, more preferably in the range of from 0.25 to 1.5 weight-%, based on the weight of the cosmetic or dermatologic or pharmaceutical composition.

7. The composition of any one of claims 2 to 6, further comprising one or more emulsifiers; wherein the one or more emulsifiers preferably are selected from the group consisting of cetearyl alcohol; ceteth-20; glyceryl stearate; steareth-20; a mixture of cetyl alcohol, glyceryl stearate, PEG-75 stearate, ceteth-20 and stearaeth-20; carbomer; xanthan gum; a mixture of sodium acrylate and sodium acryloyldimethyl taurate copolymer; a mixture of beheneth-30 phosphate, cetostearyl alcohol, dicetyl phosphate and cetyl phosphate; cetearyl olivate and sorbitan olivate; ceteareth-6 olivate and mixtures of two or more thereof; more preferably selected from the group consisting of cetearyl alcohol; ceteth-20; glyceryl stearate; steareth-20; a mixture of cetyl alcohol, glyceryl stearate, PEG-75 stearate, ceteth-20 and stearaeth-20; carbomer; xanthan gum; a mixture of sodium acrylate and sodium acryloyldimethyl taurate copolymer; a mixture of beheneth-30 phosphate, cetostearyl alcohol, dicetyl phosphate and cetyl phosphate; and mixtures of two or more thereof.

8. The composition of any one of claims 2 to 7, further comprising one or more emollients; wherein the one or more emollients preferably are selected from the group consisting of dicaprylyl carbonate; octyldodecanol; dicaprylyl ether; cyclomethicone; a mixture of isodecane, hydrogenated tetradecenyl and methylpentadecene; pentaerythrityl tetralaurate; isocetyl stearate; stearyl dimethicone; neopentyl glycol diethylhexanoate; cetyl ricinoleate; shorea robusta seed butter; and mixtures of two or more thereof; more preferably selected from the group consisting of dicaprylyl carbonate; octyldodecanol; dicaprylyl ether; cyclomethicone; a mixture of isodecane, hydrogenated tetradecenyl and methylpentadecene; pentaerythrityl tetralaurate; and mixtures of two or more thereof.

9. The composition of any one of claims 2 to 8, being for oral use; preferably the cosmetic or dermatologic or pharmaceutical composition is in the form of a liquid, a capsule, a gummy, a powder, or a gel.

10. The composition of claim 9, wherein the effective amount of the compound for use in the treatment and/or prevention of oily or oily-prone skin and/or acne is in the range of from 2 to 100 weight-%, preferably in the range of from 5 to 50 weight-%, more preferably in the range of from 5 to 40 weight-%, more preferably in the range of from 8 to 35 weight-%, based on the weight of the cosmetic or dermatologic or pharmaceutical composition.

11. The composition of claim 9 or 10, further comprising a plant-based ingredient, preferably the plant-based ingredient being one or more of green tea extract, *Desmodium* extract, nettle extract, Broccoli seed oil, chamomile extract, cucumber extract, and seaweed extract, preferably the plant-based ingredient being one or more of green tea extract, *Desmodium* extract, nettle extract and broccoli seed oil.

12. The composition of any one of claims 9 to 11, further comprising an antioxidant and/or anti-inflammatory component, preferably said component being selected from the group consisting of zinc, zinc bisglycinate, selenium, matcha tea, vitamin A, vitamin C, vitamin E, N-acetyl-cysteine, lutein, zeaxanthin, lycopene, turmeric, OPCs, OPC derivatives, glutathione, astaxanthin, and mixtures of two or more thereof, more preferably selected from the group consisting of zinc, zinc bisglycinate, selenium, matcha tea, and mixtures of two or more thereof.

13. The composition of any one of claims 9 to 12, wherein the daily dosage of the compound for use in the treatment and/or prevention of oily or oily-prone skin and/or acne being sialic acid and/or any derivatives thereof is in the range of from 10 mg/day to 1g/day, preferably in the range of from 50 mg/day to 750 mg/day, more preferably in the range of from 100 mg/day to 500 mg/day.

14. A non-therapeutical method for the treatment and/or prevention of oily or oily-prone skin and/or acne, the method comprising
applying the cosmetic or dermatologic or pharmaceutical composition of any one of claims 2 to 8 to a skin in need at least once a day, preferably twice a day; or
administering orally the cosmetic or dermatologic or pharmaceutical composition for use according to any one of claims 2 to 4 and 9 to 13 to a person in need once or twice a day.

15. Use of a cosmetic or dermatologic or pharmaceutical composition according to any one of claims 2 to 13 for inhibiting and/or treating *Cutibacterium acnes.*
